(19) Europäisches Patentamt
European Patent Office
Office européen des brevets

(11) **EP 3 764 930 B1**

(12) **EUROPÄISCHE PATENTSCHRIFT**

(45) Veröffentlichungstag und Bekanntmachung des Hinweises auf die Patenterteilung:
**06.07.2022 Patentblatt 2022/27**

(21) Anmeldenummer: **19711069.5**

(22) Anmeldetag: **12.03.2019**

(51) Internationale Patentklassifikation (IPC):
***A61B 18/12*** *(2006.01)*

(52) Gemeinsame Patentklassifikation (CPC):
**A61B 18/1206;** A61B 2018/1213

(86) Internationale Anmeldenummer:
**PCT/EP2019/056172**

(87) Internationale Veröffentlichungsnummer:
**WO 2019/175181 (19.09.2019 Gazette 2019/38)**

(54) **HOCHFREQUENZGENERATOR UND STEUEREINHEIT ZUM BETREIBEN EINES HOCHFREQUENZGENERATORS**

HIGH-FREQUENCY GENERATOR AND CONTROL UNIT FOR OPERATING A HIGH-FREQUENCY GENERATOR

GÉNÉRATEUR HAUTE FRÉQUENCE ET UNITÉ DE COMMANDE D'UN GÉNÉRATEUR HAUTE FRÉQUENCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.03.2018 DE 102018105812**

(43) Veröffentlichungstag der Anmeldung:
**20.01.2021 Patentblatt 2021/03**

(73) Patentinhaber: **Olympus Winter & Ibe GmbH 22045 Hamburg (DE)**

(72) Erfinder: **DIJKSTRA, Jelle 10781 Berlin (DE)**

(74) Vertreter: **Eisenführ Speiser Patentanwälte Rechtsanwälte PartGmbB Stralauer Platz 34 10243 Berlin (DE)**

(56) Entgegenhaltungen:
**WO-A1-02/11634      WO-A1-95/09577 WO-A1-2004/062516**

**Beschreibung**

Hochfrequenzgenerator und Steuereinheit zum Betreiben eines Hochfrequenz-generators Die Erfindung betrifft einen Hochfrequenzgenerator zum Anschließen eines elektrochirurgischen Instrumentes mit einem elektrischen Ausgangsanschluss für ein elektrochirurgisches Instrument, einem Netzteil, das wenigstens mittelbar mit dem Ausgangsanschluss verbunden ist, und einer Leistungssteuerung zum Steuern der über den Ausgangsanschluss abgegebenen elektrischen Abgabeleistung.

Hochfrequenzgeneratoren, insbesondere Hochfrequenzgeneratoren zum Versorgen elektrochirurgischer Instrumente, sind allgemein bekannt. Zum Erzeugen eines Schneidplasmas zum Schneiden von Körpergewebe ist eine hohe Zündleistung erforderlich, welche in der Regel eine zulässige Durchschnittsleistung eines Generators um ein Vielfaches übersteigt. Aus diesem Grunde ist es sinnvoll, die elektrische Abgabeleistung eines Generators zu pulsen, das heißt in Pulsen abzugeben. Durch entsprechend lange Pausen zwischen den Pulsen kann gewährleistet werden, dass trotz der hohen Zündleistung eine zulässige Durchschnittsleistung des Generators nicht überschritten wird.

WO 2010/108523 beschreibt einen Hochfrequenzgenerator zum Anschluss eines elektrochirurgischen Instrumentes für das Schneiden von Körpergewebe mittels eines Lichtbogens. Das Instrument weist eine Leistungssteuerung zum Steuern der über den Ausgangsanschluss abgegebenen elektrischen Leistung auf. Die Leistungssteuerung ist ausgebildet, zunächst für eine Phase zur Anschnittunterstützung mit vorgegebener Maximaldauer, die Abgabe einer hohen Ausgangsleistung zu bewirken und im Anschluss daran entweder, falls es während der Phase zur Anschnittunterstützung zum Zünden eines Lichtbogens gekommen ist, für eine vorbestimmte Zeitdauer einer Schnittphase die Abgabe einer gegenüber der hohen Leistung verminderten Leistung zu bewirken und daran anschließend für eine vorbestimmte Zeitdauer eines langen Pausenintervalls die Abgabe keiner oder einer geringeren Leistung zu bewirken, bei der kein Lichtbogen auftritt, oder, falls es bis zum Erreichen der vorgegebenen Maximaldauer bei der Anschnittunterstützung zu keinem Zünden eines Lichtbogens gekommen ist, für eine vorbestimmte Zeitdauer eines kurzen Pausenintervalls die Abgabe keiner oder einer geringen Leistung zu bewirken.

Ein Hochfrequenzgenerator entsprechend der Präambel des Anspruchs 1 ist aus WO 95/09577 A1 bekannt.

Der Erfindung liegt die Aufgabe zugrunde, einen verbesserten Hochfrequenzgenerator anzugeben, der bei unterschiedlichen Umgebungsbedingungen eine möglichst gleichermaßen gute Leistungssteuerung bereitstellt.

Hierzu wird erfindungsgemäß ein Hochfrequenzgenerator gemäß Anspruch 1 vorgeschlagen.

Der Hochfrequenzgenerator ist zum Anschließen eines elektrochirurgischen Instrumentes ausgebildet und weist einen elektrischen Ausgangsanschluss für ein elektrochirurgisches Instrument, einem Netzteil, das wenigstens mittelbar mit dem Ausgangsanschluss verbunden ist und eine Leistungssteuerung zum Steuern der über den Ausgangsanschluss abgegebenen elektrischen Abgabeleistung auf.

Die Leistungssteuerung ist ausgebildet, die Abgabe einer elektrischen Abgabeleistung zu beginnen, wenn eine Abgabeenergie-Bilanzmenge größer als ein Abgabeenergie-Grenzwert ist, wobei die Abgabeenergie-Bilanzmenge über eine gleitende Bestimmungsdauer aus einer zugeführten vorgegebenen Generatorleistung und der abgegebenen elektrischen Abgabeleistung bestimmt wird.

Die Erfindung basiert auf der Überlegung, dass - insbesondere hinsichtlich des genannten Ansatzes des Standes der Technik - die Bestimmung einer geeigneten elektrischen Abgabeleistung noch verbessert werden kann. Mit dem Begriff "Bestimmen" der Abgabeenergie-Bilanzmenge ist das Bilden eines Wertes gemeint, der die tatsächliche Bilanz aus einer vorgegebenen Generatorleistung und der abgegebenen elektrischen Abgabeleistung möglichst genau, zumindest aber für eine Steuerung ausreichend genau, wiedergibt. Der Begriff "vorgegebene Generatorleistung" bezeichnet dabei eine durch den Benutzer einstellbare, vom Generator über die Elektrode abzugebende, über die Bestimmungszeitdauer gemittelte Leistung.

Ein erster Aspekt betrifft die Bestimmung von geeigneten Pulsdauern und den dazwischenliegenden Pausendauern. Insbesondere im eingangs genannten Ansatz, in dem die Pulsdauer und/oder die Pausendauer als starre Zeitfenster festgelegt werden, kann der Fall eintreten, dass nach Ablauf der Pulsdauer die Abgabe eines Energiepulses beendet wird, obwohl gerade erfolgreich ein Plasma, entsteht.

Auch kann bei dem aus dem Stand der Technik bekannten Ansatz der Fall eintreten, dass die starren Pausendauern tendenziell zu lang gewählt werden, um sicherzustellen, dass eine gesetzliche und/oder normative vorgeschriebene maximal zulässige Energiemenge innerhalb einer Bilanzdauer nicht überschritten wird, oder Pausen häufiger als nötig auftreten. Dies führt dazu, dass potentiell verfügbare Energiereserven nicht genutzt werden.

Die Erfindung schließt die Erkenntnis ein, dass durch eine flexible, insbesondere den Umgebungsbedingungen angepasste Steuerung der elektrischen Abgabeleistung vorhandene Energiereserven besser genutzt werden können, ohne eine maximal zulässige Generatorleistung zu überschreiten. Insbesondere können Impulse mit einer Zündleistung zum Zünden eines Plasmas bei Bedarf derart gesteuert werden, dass sie länger dauern, solange eine aus einer maximal zulässigen Generatorleistung resultierende Energiemenge nicht überschritten wird - dies ist insbesondere vorteilhaft im Vergleich zu einer starren Pulsdauer.

Ein weiterer Aspekt betrifft die Möglichkeit einer besseren Kontrolle der Abgabeenergie. Eine rein zeitliche Steuerung der Abgabeenergie - wie im eingangs genannten Stand der Technik offenbart - führt zu einer Gefahr eines unkontrollierten, wiederholten Umschaltens zwischen einer Zündleistung und einer Schneidleistung, was insbesondere bei einem instabilen Schneidplasma oder bei einer zu niedrig vorgegebenen Generatorleistung auftreten kann. In diesem Falle kann das wiederholte Abgeben der hohen Zündleistung zu einem unkontrolliert hohen Energieeintrag in das Gewebe führen.

Durch eine Abgabeenergie-Bilanzmenge kann im Sinne eines virtuellen Energiespeichers mit Berücksichtigung von dessen Entleerung durch eine tatsächlich abgegebene Energiemenge erfasst werden, wie viel Energie in einer zurückliegenden, gleitenden Bestimmungsdauer bereits verbraucht wurde, und folglich, wie viel Energie unter den festgelegten Bestimmungen momentan noch verfügbar ist. Somit kann vorteilhaft die Gefahr eines unkontrolliert hohen Energieeintrags in das zu behandelnde Gewebe verringert oder sogar minimiert werden, ohne eine starre zeitliche Beschränkung der Energieabgabe vorzugeben.

"Gleitend" bedeutet in diesem Zusammenhang, dass die Bestimmungsdauer als Bilanzrahmen eine nicht veränderliche Dauer aufweist, sich jedoch mit fortlaufender Zeit bewegt, wodurch ein rollierendes Zeitfenster entsteht, in dem die Abgabeenergie-Bilanzmenge iterativ, beispielsweise in jedem neuen Programmzyklus oder pro festgelegter Zyklusdauer, neu berechnet wird. Bei der Berechnung wird insbesondere eine vorgegebene Generatorleistung und eine - tatsächlich abgegebene - elektrische Abgabeleistung bilanziert. Im übertragenen Sinne wird folglich einem durch eine Bestimmungsdauer und eine maximale Abgabeenergie-Bilanzmenge definierten virtuellen Energiespeicher sowohl Energie gemäß einer vorgegebenen Generatorleistung zugeführt, als auch Energie gemäß einer elektrischen Abgabeleistung entnommen. Durch die vorgegebene Generatorleistung wird bestimmt, wie viel Energie, dem virtuellen Energiespeicher zugeführt wird, während ihm gleichzeitig, die tatsächlich abgegebene elektrische Abgabeleistung entnommen wird. Die daraus resultierende Differenz ist die Abgabeenergie-Bilanzmenge.

Vorzugsweise ist vorgesehen, dass die vorgegebene Generatorleistung einstellbar ist. Dies beinhaltet konkret, dass der Wert der vorgegebenen Generatorleistung, insbesondere ein skalarer Zahlenwert mit der Einheit Watt, über eine geeignete Benutzerschnittstelle oder ein Bedienelement eingestellt werden kann. Beispielsweise kann ein solches Bedienelement als Drehregler oder als Anordnung von Pfeiltasten gebildet sein, und optional über eine Anzeige verfügen, in der der aktuell eingestellte Wert der vorgegebenen Generatorleistung angezeigt wird. Durch eine Einstellbarkeit der vorgegebenen Generatorleistung kann vorteilhaft die Abgabe von elektrischer Abgabeenergie beeinflusst und somit an konkrete Anwendungen angepasst werden. Hierbei sind gegebenenfalls, insbesondere als obere Leistungsgrenze, gesetzliche und/oder normative Vorgaben zu berücksichtigen.

Vorzugsweise ist vorgesehen, dass die Abgabeenergie-Bilanzmenge eine maximale Abgabeenergie-Bilanzmenge nicht überschreitet. Dies beinhaltet konkret, dass die Abgabeenergie-Bilanzmenge begrenzt ist und - im übertragenen Sinne - der virtuelle Energiespeicher ein maximales Fassungsvermögen aufweist, ab dem keine weitere Energie mehr gespeichert wird. Durch eine maximale Abgabeenergie-Bilanzmenge kann erreicht werden, dass insbesondere unter Berücksichtigung einer vorgegebenen Generatorleistung gesetzliche und/oder normative Vorgaben eingehalten werden. Diese Vorgaben betreffen insbesondere eine maximal zulässige abzugebende Energiemenge innerhalb eines Bestimmungszeitraums. Insbesondere ist ein solcher Rahmen in der Richtlinie DIN EN 60601-2-2 mit dem Titel "Medizinische elektrische Geräte - Teil 2-2: Besondere Festlegungen für die Sicherheit von Hochfrequenz-Chirurgiegeräten" vorgegeben, die beschreibt, dass innerhalb eines Bestimmungszeitraums von einer Sekunde eine maximal zulässige abzugebende Energiemenge von 400 J nicht überschritten wird. In einer derartigen Weiterbildung kann vorteilhaft das Risiko eines Überschreitens einer maximalen Energiemenge, insbesondere zur Gewährleistung der Patientensicherheit, verringert werden. In möglichen Weiterbildungen kann die maximale Abgabeenergie-Bilanzmenge noch um einen bestimmten Betrag, beispielsweise um zehn Prozent der maximalen Abgabeenergie-Bilanzmenge, geringer gewählt werden, um einen Sicherheitspuffer vorzuhalten, beispielsweise um das Risiko eines Überschreitens gesetzlicher und/oder normativer Vorgaben weiter zu verringern.

Im Rahmen einer Weiterbildung ist vorgesehen, dass die maximale Abgabeenergie-Bilanzmenge aus der Differenz aus einer zulässigen Energiemenge und einer abgegebenen Energiemenge bestimmt wird, wobei die zulässige Energiemenge die Energiemenge ist, die in einer Bilanzdauer maximal abgegeben werden darf. Vorgaben hinsichtlich der zulässigen Energiemenge und der Bilanzdauer können sich aufgrund gesetzlicher und/oder normativer Bestimmungen ergeben. Hierbei ermöglicht die Weiterbildung in Zusammenhang mit einer Abgabeenergie-Bilanzmenge, dass ein Einhalten gesetzlicher und/oder normativer Vorgaben erreicht wird, und trotzdem stets die - innerhalb dieser Vorgaben - maximal verfügbare Abgabeleistung genutzt wird. Dies ist insbesondere im Gegensatz zum Stand der Technik zu sehen, wo ein Einhalten derartiger Vorgaben insbesondere durch festgelegte, konservativ ausgelegte Pausendauern zwischen Abgabeenergie-, insbesondere Zündpulsen, erreicht wird - und dadurch potentiell verfügbare Leistung nicht genutzt wird. In bevorzugten Weiterbildungen kann die Bilanzdauer gleich der Bestimmungsdauer sein.

Der Abgabeenergie-Grenzwert kann einstellbar sein. Mittels der Einstellbarkeit des Abgabeenergie-Grenzwertes kann vorteilhaft das Verhalten der Leistungssteuerung - und somit das Schneidverhalten des Hochfrequenzgenerators - beeinflusst werden. Ein niedriger Abgabeenergie-Grenzwert führt dazu, dass dem Hochfrequenzgenerator nach einem Zeitpunkt, an dem die Abgabeenergie-Bilanzmenge gleich null ist - das heißt der Energiespeicher vollständig entleert wurde - wieder schneller eine benötigte Energiemenge zur Abgabe einer Abgabeleistung, insbesondere eine Zündleistung, zur Verfügung steht. Jedoch hat ein niedriger Abgabeenergie-Grenzwert auch zur Folge, dass die Abgabeenergie-Bilanzmenge wieder schneller den Minimalwert unterschreitet, insbesondere auf null zurückfällt. Auch gilt, dass wenn der Abgabeenergie-Grenzwert zu niedrig gewählt wird, die im Speicher verfügbare Energie nicht ausreicht, ein Plasma zu zünden. Wird der Abgabeenergie-Grenzwert hingegen größer gewählt, ist nach Erreichen des Abgabeenergie-Grenzwerts mehr Abgabeenergie - insbesondere für längere Zündpulse - verfügbar. Allerdings sind in diesem Fall auch die Pausendauern, das heißt die Wartezeit zum Erreichen des Abgabeenergie-Grenzwerts, entsprechend länger.

Im Rahmen einer Weiterbildung ist vorgesehen, dass die Leistungssteuerung eine Bilanzierungseinheit aufweist, die zum zyklischen Bestimmen der Abgabeenergie-Bilanzmenge ausgebildet ist. Dies beinhaltet konkret, dass die Bilanzierungseinheit durch zyklisches Neuberechnen der Bilanzmenge den virtuellen Energiespeicher stets aktualisiert. Hierbei erfolgt das zyklische Neuberechnen innerhalb einer Zykluszeit, welche kürzer als die Bestimmungsdauer ist. In möglichen Weiterbildungen ist die Zykluszeit mindestens um den Faktor 100, bevorzugt um den Faktor 1000, besonders bevorzugt um den Faktor 10.000 kürzer als die Bestimmungsdauer. Beispielsweise kann, bei einer Bestimmungsdauer von 1 Sekunde, für die Zykluszeit ein Wert zwischen 100 $\mu$s und 5 ms gewählt werden. Durch eine kurze Zykluszeit steht der Leistungssteuerung vorteilhaft stets ein aktueller Wert der Abgabeenergie-Bilanzmenge zur Verfügung, sodass eine Regelung der elektrischen Abgabeleistung auf Basis der Abgabeenergie-Bilanzmenge mit vernachlässigbarer Verzögerung möglich ist.

Zur Lösung der eingangs genannten Aufgabe wird außerdem eine Leistungssteuerung für einen Hochfrequenzgenerator gemäß Anspruch 7 vorgeschlagen. Der Hochfrequenzgenerator weist hierbei einen elektrischen Ausgangsanschluss für ein elektrochirurgisches Instrument, eine Strom- oder Spannungsquelle, die wenigstens mittelbar mit dem Ausgangsanschluss verbunden ist, und eine Leistungssteuerung auf. Die Leistungssteuerung ist ausgebildet, die Abgabe einer elektrischen Abgabeleistung zu beginnen, wenn eine Abgabeenergie-Bilanzmenge größer als ein Abgabeenergie-Grenzwert ist oder einen Initialwert aufweist, und die Abgabe der elektrischen Abgabeleistung zu beenden, wenn die Abgabeenergie-Bilanzmenge einen Minimalwert unterschreitet, wobei die Abgabeenergie-Bilanzmenge über eine gleitende Bestimmungsdauer bestimmt wird, wobei die Abgabeenergie-Bilanzmenge als eine Differenz aus einer vorgegebenen Generatorleistung und der elektrischen Abgabeleistung gebildet wird. Bei der erfindungsgemäßen Leistungssteuerung werden die Vorteile des erfindungsgemäßen Hochfrequenzgenerators vorteilhaft genutzt. In möglichen Weiterbildungen der Leistungssteuerung kann diese derart ausgebildet sein, dass sie sich zur Nachrüstung in bestehende Hochfrequenzgeneratoren eignet. Hierzu kann die Leistungssteuerung als - insbesondere austauschbares - Hardware- und/oder Softwaremodul ausgebildet sein.

Zur Lösung der eingangs genannten Aufgabe wird außerdem ein Verfahren zum Betreiben eines Hochfrequenzgenerators mit einer Leistungssteuerung vorgeschlagen, das jedoch nicht unter den Schutzumfang der Ansprüche fällt. Das Verfahren umfasst folgende Schritte:

- Initialisieren einer Abgabeenergie-Bilanzmenge auf eine Initialenergie; und
- selektives Beginnen einer Abgabe einer elektrischen Abgabeleistung, insbesondere durch Betätigen eines Fußschalters, wenn eine Abgabeenergie-Bilanzmenge größer als ein Abgabeenergie-Grenzwert ist oder eine Initialenergie aufweist.

In einer Weiterbildung des Verfahrens, die ebenfalls nicht Teil der Erfindung ist, ist vorgesehen, dass die Abgabe der elektrischen Abgabeleistung beendet wird, wenn die Abgabeenergie-Bilanzmenge gleich null ist.

In einer Weiterbildung des Verfahrens, die ebenfalls nicht Teil der Erfindung ist, ist vorgesehen, dass die Abgabeenergie-Bilanzmenge über eine gleitende Bestimmungsdauer aus einer zugeführten vorgegebenen Generatorleistung und der abgegebenen elektrischen Abgabeleistung bestimmt wird.

Beim Verfahren zum Betreiben eines Hochfrequenzgenerators werden die Vorteile des Hochfrequenzgenerators vorteilhaft genutzt.

Weitere Vorteile, Merkmale und Einzelheiten der Erfindung ergeben sich aus der nachfolgenden Beschreibung der bevorzugten Ausführungsformen sowie anhand der Zeichnung; diese zeigt in:

Fig. 1: einen Hochfrequenzgenerator, mit einem angeschlossenen elektrochirurgischen Instrument;

Fig. 2: ein Prinzipschaltbild eines Hochfrequenzgenerators gemäß dem Konzept der Erfindung;

Fig. 3A: eine schematische Darstellung der Bestimmung einer maximalen Abgabeenergie-Bilanzmenge;

Fig. 3B: ein Zustandsdiagramm für einen möglichen Betrieb des Hochfrequenzgenerators;

Fig. 3C: ein Verlaufsdiagramm mit schematischen Verläufen der Abgabeleistung und der Abgabeenergie-Bilanzmenge;

Fig. 4A, B: ein detailliertes Prinzipschaltbild eines Aktivators und mögliche Schaltzustände eines Einschaltmoduls.

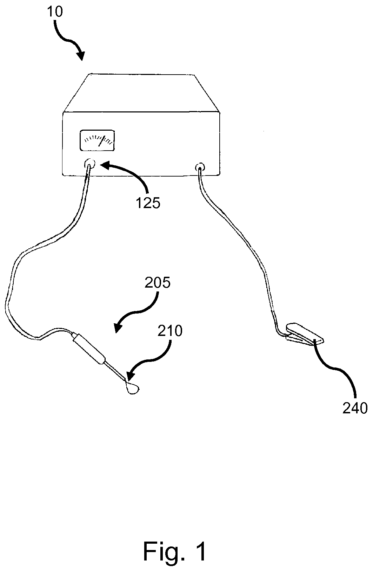

Fig. 1 zeigt einen Hochfrequenzgenerator 10, welcher eingangsseitig mit einem Stromkabel zum Anschluss an ein Stromnetz versehen ist. Der Hochfrequenzgenerator 10 ist mit einem Fußschalter 240 verbunden, der zum Aktivieren des Hochfrequenzgenerators 10 dient. Ausgangsseitig sind zwei hier nicht näher dargestellte Ausgangspole, ein erster Ausgangspol 125.1 und ein zweiter Ausgangspol 125.2, eines Ausgangsanschlusses 125 des Hochfrequenzgenerators 10 über Stromleitungen mit einer bipolaren Elektrode 210 eines elektrochirurgischen Instruments 205 verbunden. Das elektrochirurgische Instrument verfügt dabei über eine bipolare Schneidelektrode 210, die in der beispielhaften Ausführungsform gemäß Fig. 1 als bipolares Schlaufenschneidinstrument ausgebildet ist.

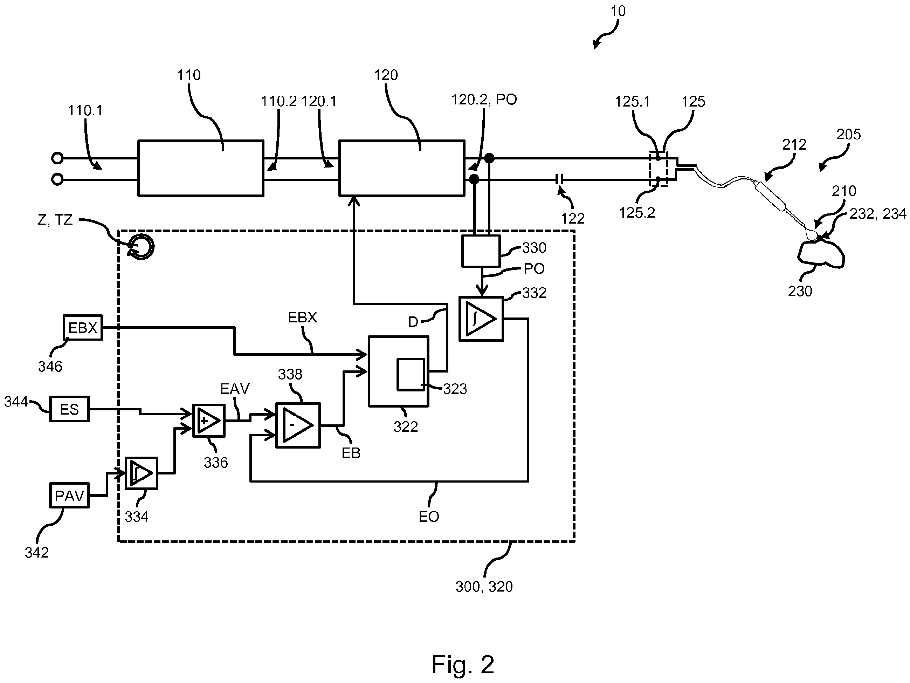

Fig. 2 zeigt das Prinzipschaltbild eines bevorzugten Ausführungsbeispiels des erfindungsgemäßen Hochfrequenzgenerators 10 mit einem gegenüber dem Stand der Technik verbesserten Anschnittverhalten. Dargestelltes Ausführungsbeispiel ist für chirurgische Eingriffe insbesondere auf dem Gebiet der transurethralen Resektion (TURis), aber auch auf anderen Gebieten wie zum Beispiel der Endoskopischen Mukosa Resektion (EMR) und der Polypektomie, geeignet.

Der Hochfrequenzgenerator 10 enthält ein Netzteil 110, welches an einem Eingang 110.1 mit einem Wechselstromversorgungsnetz verbindbar ist und ausgangsseitig an einem Ausgang 110.2 mit einem Eingang 120.1 eines taktsteuerbaren Hochfrequenzgeneratormoduls 120 verbunden ist. Das Netzteil 110 wandelt eine Wechselspannung in eine Gleichspannung um.

Das Hochfrequenzgeneratormodul 120 wandelt die Gleichspannung in eine Wechselspannung mit einer Frequenz zwischen 0,3 und 2 MHz um. Ein Ausgang 120.2 des Hochfrequenzgeneratormoduls 120 ist über mindestens einen Antifaradisationskondensator 122 und über einen zweipoligen elektrischen Ausgangsanschluss 125 des Hochfrequenzgenerators 10 mit einer bipolaren Elektrode 210 eines elektrochirurgischen Instrumentes 205 verbunden. Der mindestens eine Antifaradisationskondensator soll die Übertragung von Gleichströmen verhindern. Die Abgabe der Wechselspannung am Ausgang 120.2 erfolgt dabei in taktgesteuerter Weise. "Taktgesteuert" bedeutet, dass das Hochfrequenzgeneratormodul 120 mittels eines Aktivators 322 so getaktet werden kann, dass am Ausgang 120.2 Impulsfolgen mit Impulsen unterschiedlicher Impulslängen und unterschiedlicher Impulsverläufe zur Verfügung gestellt werden. Die Impulsamplitude kann sich über den Verlauf des Impulses ändern. Die Leistungsabgabe des Hochfrequenzgeneratormoduls 120 ist zeitlich gesteuert derart, dass in Abhängigkeit einer Abgabeenergie-Bilanzmenge EB eine Abfolge einer elektrischen Abgabeleistung unterschiedlicher Leistungsniveaus und Dauern abgegeben werden kann.

Das elektrochirurgische Instrument 205 besteht in der Regel aus einer Schneidelektrode 210 und einem Griffteil 212. Um ein in der Nähe der Schneidelektrode 210 befindliches - hier ausschnittsweise dargestelltes - Gewebe 230 elektrochirurgisch schneiden zu können, kann an dem Körpergewebe 230 der zu operierende Bereich mit einer Kochsalzlösung 232 beaufschlagt und in diesem Bereich durch die Elektrode 210 ein Plasma 234 erzeugt werden. Dies geschieht dadurch, dass am Ausgang 120.2 des Hochfrequenzgeneratormoduls 120 ein Hochfrequenzstrom mit hoher elektrischer Leistung, insbesondere eine Zündleistung PI, ausgegeben wird. An den Ausgang 120.2 des Hochfrequenzgeneratormoduls 120 ist über den zweipoligen elektrischen Ausgangsanschluss 125 die bipolare Schneidelektrode 210 des elektrochirurgischen Instruments angeschlossen. Durch den an die bipolare Schneidelektrode 210 ausgegebenen Hochfrequenzstrom erfolgt eine Erwärmung der Kochsalzlösung 232 im Bereich der bipolaren Schneidelektrode 210, sodass das Plasma 234 gezündet wird. Durch das Plasma 234 ist der Operateur in der Lage, einen gewünschten Schnitt im an der Schneidelektrode 210 anliegenden Gewebe 230 durchzuführen. Den Schneidvorgang kann der Operateur allgemein durch Betätigen eines hier nicht dargestellten Fußschalters 240 auslösen und beenden.

Um das für das elektrochirurgische Schneiden erforderliche Plasma aufrecht zu erhalten, ist eine vergleichsweise niedrigere elektrische Leistung, insbesondere eine Schneidleistung PC, ausreichend. Dies ist der Fall, da das Plasma einen wesentlich höheren elektrischen Widerstand als die Saline aufweist. Der elektrische Widerstand des Plasmas kann im Bereich mehrerer hundert Ohm liegen, wohingegen der elektrische Widerstand der Saline beispielsweise im Bereich von ca. 25 Ohm liegen kann.

Der Hochfrequenzgenerator 10 weist eine Leistungssteuerung 300 auf, die wiederum eine Bilanzierungseinheit 320 umfasst.

Die Bilanzierungseinheit 320 weist eine Leistungserfassungseinheit 330 auf. Die Leistungserfassungseinheit 330 ist mit dem Ausgang 120.2 des Hochfrequenzgeneratormoduls 120 derart verbunden, dass eine tatsächlich abgegebene elektrische Abgabeleistung PO erfasst, insbesondere gemessen, werden kann. Der Ausgang der Leistungserfassungseinheit 330 ist mit einem ersten Integrator 332 verbunden. Der erste Integrator 332 ist ausgebildet, den Wert, insbesondere Messwert, der elektrischen Abgabeleistung PO über eine Bestimmungsdauer TA zu integrieren, um die innerhalb dieser Bestimmungsdauer TA vom Hochfrequenzgeneratormodul 120 tatsächlich abgegebene AbgabeEnergiemenge EO zu ermitteln.

Die Bilanzierungseinheit 320 weist weiterhin einen zweiten Integrator 334 auf. Dieser ist dazu ausgebildet, einen Wert für eine vorgegebene Generatorleistung PAV über die Bestimmungsdauer TA zu integrieren. Der zweite Integrator 334 ist eingangsseitig mit einem Generatorleistung-Einstellmodul 342 verbunden, mittels welchem eine vorgegebene Generatorleistung PAV vom Benutzer eingestellt werden kann. Der zweite Integrator 334 überträgt ausgangsseitig das Ergebnis der Integration an einen Summenbilder 336 der zu dem Integral der vorgegebenen Generatorleistung eine Initialenergie ES addiert und so eine vorgegebene Energiemenge EAV bestimmt. In bestimmten Ausführungsformen kann alternativ die vorgegebene Energiemenge EAV vereinfacht gebildet werden, indem die vorgegebene Generatorleistung PAV mit der Bestimmungsdauer TA multipliziert wird. In diesem Falle gilt: EAV = PAV * TA.

Die von dem Summenbilder 336 zu dem Integral der vorgegebenen Generatorleistung zu addierende Initialenergie ES ist über ein Initialenergie-Einstellmodul 344 einstellbar. Hierbei ist die Initialenergie ES insbesondere so zu wählen, dass eine maximale Abgabeenergie-Bilanzmenge EBMAX nicht überschritten wird.

In einem Differenzbilder 338 wird die Abgabe-Energiemenge EO von der vorgegebenen Energiemenge EAV abgezogen, um die Abgabeenergie-Bilanzmenge EB zu bilden. Hieraus ergibt sich der Formelmäßige Zusammenhang:

$$EB = ES + \int_{TA} PAV - \int_{TA} PO = EAV - EO$$

Ein Aktivator 322 ist eingangsseitig mit dem Differenzbilder 338 und mit einem Grenzwert-Einstellmodul 346 verbunden. Über das Grenzwert-Einstellmodul 346 kann ein Abgabeenergie-Grenzwert EBX eingestellt werden. Der Abgabeenergie-Grenzwert EBX definiert insbesondere den Minimalbetrag einer steigenden Abgabeenergie-Bilanzmenge EB, ab dem die Abgabe einer elektrischen Abgabeleistung PO, insbesondere einer Zündleistung PI, erfolgt. Nur wenn somit mehr als eine durch den Abgabeenergie-Grenzwert EBX definierte Mindestenergiemenge in dem virtuellen Energiespeicher vorhanden ist wird somit eine Zündleistung abgegeben. In Abhängigkeit der eingehenden Größen, nämlich der Abgabeenergie-Bilanzmenge EB und dem Abgabeenergie-Grenzwert EBX, gibt der Aktivator 322 ein Einschaltsignal D aus. Zur Übertragung des Einschaltsignals D an das Hochfrequenzgeneratormodul 120 ist der Aktivator 322 ausgangsseitig mit dem Hochfrequenzgeneratormodul 120 signalführend verbunden. Über das Einschaltsignal D kann die Abgabe einer Abgabeenergie PO durch das Hochfrequenzgeneratormodul 120 aktiviert und deaktiviert, das heißt begonnen und beendet, werden.

Die Bilanzierungseinheit 320 arbeitet in mit einer Zykluszeit TZ, das bedeutet, dass die oben beschriebenen Berechnungen zum Bilden der Abgabeenergie-Bilanzmenge EB nach einem Zyklus T mit einer Zykluszeit TZ mit aktualisierten Eingangsdaten, insbesondere der aktuellen vorgegebenen Generatorleistung PAV und der aktuellen Abgabeleistung PO, in einem neuen Zyklus T+1 erneut durchgeführt werden. Somit werden die Werte für die abgegebene Energiemenge EO und die vorgegebene Energiemenge EAV mit jedem Zyklus T aktualisiert. Die Zykluszeit TZ kann insbesondere abhängig von der konkreten Ausbildung der Bilanzierungseinheit sein, insbesondere von der verwendeten Hard- und Software, und beispielsweise zwischen 100 μs und 5 ms betragen.

Die Leistungssteuerung 300 kann auch ganz oder teilweise in einem Mikrokontroller realisiert sein, in dem die Komponente 320 und optional weitere Komponenten programmiert sind.

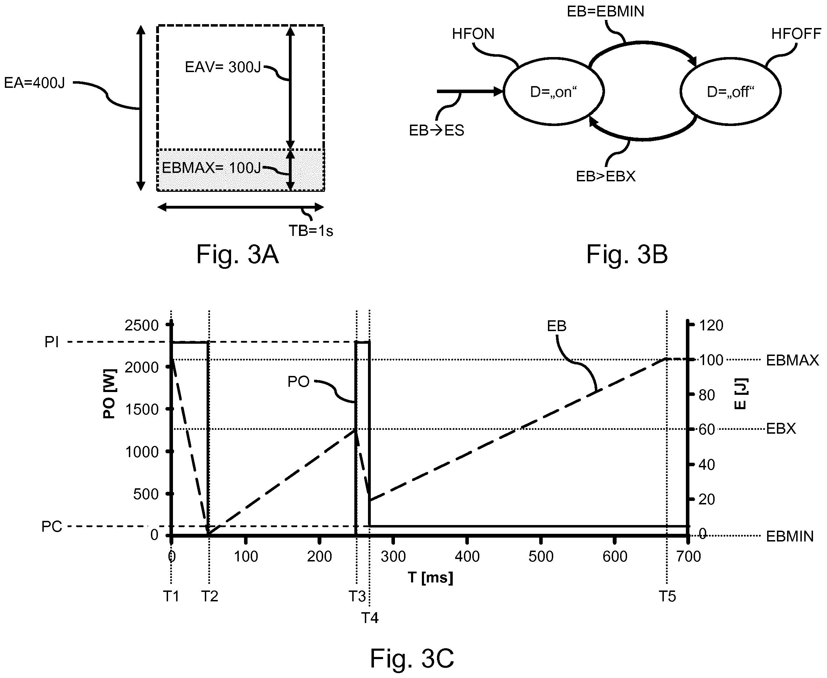

Fig. 3A zeigt schematisch ein Diagramm zum Ermitteln einer maximalen Abgabeenergie-Bilanzmenge EBMAX und somit - im übertragenen Sinne - zur Ermittlung der Größe des Energiespeichers. Der äußere Rahmen, hier gestrichelt dargestellt, wird gebildet durch technische und/oder gesetzliche und/oder normative Vorgaben. Ein solcher Rahmen kann durch die Vorgabe aus der DIN EN 60601-2-2 mit dem Titel "Medizinische elektrische Geräte - Teil 2-2: Besondere Festlegungen für die Sicherheit von Hochfrequenz-Chirurgiegeräten" sein, die festlegt, dass innerhalb einer Bilanzdauer TB von einer Sekunde, eine zulässige Energiemenge EA von 400 J nicht überschritten werden darf. Eine einstellbare vorgegebene Generatorleistung PAV, für welche hier beispielhaft PAV=300 W gewählt ist, führt - innerhalb der Bilanzdauer TB von einer Sekunde - entsprechend zu einer Abgabe einer vorgegebenen Energiemenge EAV von 300 J. Aus der Differenz der zulässigen Energiemenge EA und der vorgegebenen Energiemenge EAV resultiert die maximale Abgabeenergie-Gesamtmenge EBMAX, welche vorliegend 100 J beträgt.

Fig. 3B zeigt ein Zustandsdiagramm für einen möglichen Betrieb des Hochfrequenzgenerators. Nach dem Aktivieren bzw. Initialisieren der Abgabeenergie-Bilanzmenge EB mit einer Initialenergie ES befindet sich der Hochfrequenzgenerator 10 in einem aktivierten Zustand HFON. In diesem aktivierten Zustand HFON hat das Einschaltsignal D den Wert "on", folglich gibt das Hochfrequenzgeneratormodul 120 eine elektrische Abgabeleistung PO ab. Wenn die Abgabeenergie-Bilanzmenge EB unterhalb eines Minimalwertes EBMIN ist, beispielsweise null beträgt - das heißt wenn der virtuelle Energiespeicher vollständig entleert ist - wechselt der Hochfrequenzgenerator 10 in einen deaktivierten Zustand HFOFF. Im deaktivierten Zustand HFOFF hat das Einschaltsignal D den Wert "off", folglich gibt das Hochfrequenzgeneratormodul 120 keine elektrische Abgabeleistung PO ab. In diesem deaktivierten Zustand HFOFF steigt die Abgabeenergie-Bilanzmenge EB, insbesondere durch den Eintrag der vorgegebenen Generatorleistung PAV, wieder an; das heißt, der virtuelle Energiespeicher lädt sich wieder auf. Sobald die Abgabeenergie-Bilanzmenge EB einen Abgabeenergie-Grenzwert EBX überschritten hat, wechselt der Hochfrequenzgenerator 10 wieder in den aktivierten Zustand HFON. Eine hohe Initialenergie ES, die maximal den Wert der maximalen Abgabeenergie-Bilanzmenge EBMAX annehmen kann, führt zu einer möglichst langen Abgabedauer nach der Aktivierung. Es kann im Rahmen der Erfindung allgemein vorteilhaft sein, für den Wert für die Initialenergie ES den gleichen Wert wie für den Abgabeenergie-Grenzwert EBX zu wählen. Auf diese Weise entspricht der erste Zündversuch nach der Initialisierung, insbesondere hinsichtlich der Pulsdauer und der darauffolgenden Wartedauer, den folgenden Zündversuchen, die jeweils nach Erreichen des Abgabeenergie-Grenzwerts EBX erfolgen. Es kann vorgesehen sein, dass die Initialenergie ES nur einmalig, zu Beginn einer Behandlung, eingebracht wird.

Fig. 3C zeigt ein Diagramm mit einem zeitlichen Verlauf einer elektrischen Abgabeleistung PO sowie einer Abgabeenergie-Bilanzmenge EB einer Weiterbildung eines Hochfrequenzgenerators 10 gemäß dem Konzept der Erfindung. Zu einem ersten Zeitpunkt T1 beträgt die elektrische Abgabeenergie PO den Wert einer Zündleistung PI, welcher vorliegend 2300 W beträgt. Eine festlegbare vorgegebene Generatorleistung PAV beträgt vorliegend 300 W und die maximale Abgabeenergie-Bilanzmenge EBMAX beträgt - wie in Fig. 3A dargestellt - 100J.

Durch die Abgabeenergie-Bilanzmenge EB kann gewährleistet werden, dass - innerhalb einer Bestimmungsdauer TA - eine bestimmte Abgabeenergiemenge, nämlich eine maximale Abgabeenergie-Bilanzmenge EBMAX nicht überschritten wird. Hierbei kann die Abgabeleistung PO kurzzeitig, insbesondere während eines Zeitraums innerhalb der Bestimmungsdauer TA, höher sein als eine durchschnittliche Abgabeleistung, die sich aus dem Quotienten aus dieser maximalen Abgabeenergie-Bilanzmenge EBMAX und der Bestimmungsdauer TA ergibt. Jedoch führt eine höhere Abgabeleistung PO dazu, dass sich die Abgabeenergie-Bilanzmenge EB schneller verringert, als neue Energie gemäß der maximalen virtuellen Abgabeleistung hinzugefügt werden kann. In anderen Worten - im Sinne eines virtuellen Energiespeichers - die Abgabeenergie-Bilanzmenge EB entlädt sich schneller, als sie aufgeladen wird.

An einem Zeitpunkt T2 ist die Abgabeenergie-Bilanzmenge EB gleich der minimalen Abgabeenergie-Bilanzmenge EBMIN, das heißt der virtuelle Energiespeicher ist erschöpft. Die minimalen Abgabeenergie-Bilanzmenge EBMIN beträgt vorliegend gleich null. Die am Zeitpunkt T1 begonnene Pulsdauer des Zündpulses ist somit am Zeitpunkt T2 beendet. Da die vorgegebene Generatorleistung PAV 300W, und die Zündleistung PI 2300W beträgt, beträgt die Differenzleistung 2000W. Bei einer maximalen Abgabeenergie-Bilanzmenge EBMAX von 100J (sowie einer anfänglichen Initialenergie ES von 100 J) ist der Zeitpunkt T2 nach 0,05 s, also 50 ms erreicht. Zum Zeitpunkt T2 wird die Abgabe einer elektrischen Abgabeenergie PO durch das Hochfrequenzgeneratormodul 120 beendet, das heißt der Hochfrequenzgenerator 10 wird in den deaktivierten Zustand HFOFF umgeschaltet. Folglich sinkt im Diagramm der Wert PO von der Zündleistung PI stufenartig auf null ab. Ab diesem Zeitpunkt T2 beginnt die Abgabeenergie-Bilanzmenge EB zu steigen, und zwar mit einer Rate, die der vorgegebenen Generatorleistung PAV = 300 W = 300 J/s entspricht. An einem dritten Zeitpunkt T3 hat die Abgabeenergie-Bilanzmenge EB den Wert des Abgabeenergie-Grenzwerts EBX erreicht, der vorliegend 60 J beträgt. Folglich liegt der dritte Zeitpunkt T3 0,2 s = 200 ms hinter dem zweiten Zeitpunkt D2 und es gilt T3=250 ms. Der Zeitraum zwischen dem Zeitpunkt T2 und dem Zeitpunkt T3 ist eine Wartedauer, in der keine elektrische Abgabeenergie PO abgegeben wird.

Ab dem Zeitpunkt T3, also nach Erreichen des Abgabeenergie-Grenzwertes EBX, wird das Hochfrequenzgeneratormodul 120 derart über ein Einschaltmodul 323 angesteuert, dass wieder die Abgabe einer elektrischen Abgabeleistung PO erfolgt. Aufgrund des niedrigen Ausgangswiderstands an der bipolaren Elektrode nimmt die elektrische Abgabeleistung PO einen relativ hohen Wert, nämlich in Höhe der Zündleistung PI an. Zu einem Zeitpunkt T4 wurde erfolgreich ein Plasma gezündet, woraufhin der Ausgangswiderstand an der bipolaren Elektrode steigt, und folglich die Abgabeleistung PO sprungartig auf einen niedrigeren Wert, insbesondere auf eine Schneidleistung PC, abfällt, wobei die Schneidleistung PC um ein Vielfaches niedriger ist als die Zündleistung PI. Vorliegend beträgt die Schneidleistung PC 100 W.

Aufgrund der Tatsache, dass die nun abgegebene Schneidleistung PC niedriger ist als die vorgegebene Generatorleistung PAV, steigt die Abgabeenergie-Bilanzmenge EB ab dem vierten Zeitpunkt T4. Im übertragenen Sinne lädt sich der virtuelle Energiespeicher also schneller auf, als er entleert wird. Dies hat zur Folge, dass die Abgabeenergie-Bilanzmenge EB an einem fünften Zeitpunkt T5 seinen Maximalwert, nämlich die maximale Abgabeenergie-Bilanzmenge EBMAX erreicht hat, die vorliegend 100 J beträgt. Bei einer Schneidleistung PC von 100 W und einer vorgegebenen Generatorleistung PAV von 300 W beträgt die Differenzleistung 200 W = 200 J/s.

Mit einem elektrochirurgischen Instrument 205 kann ein Schneiden über den Zeitpunkt T5 hinaus fortgeführt werden, entweder bis der Schnittvorgang über ein Lösen des Fußschalters 240 abgebrochen wird, oder das Plasma unterbrochen wird. Ab diesem, hier nicht dargestellten, Zeitpunkt kann ein Vorgehen wie hier beschrieben zum Anschnitt wiederholt werden.

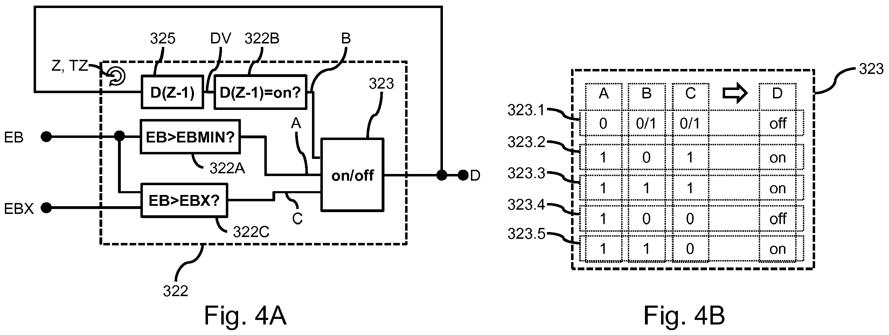

Fig. 4A zeigt in einem Prinzipschaltbild einen möglichen Aufbau eines Aktivators 322. Der Aktivator 322 weist vorliegend drei Vergleichsmodule 322A-C auf, sowie einen Zyklusspeicher 325 und ein Einschaltmodul 323. Vorliegend wird die Funktionsweise des Aktivators 322 während eines Zyklus Z veranschaulicht.

In einem ersten Vergleichsmodul 322A wird geprüft, ob der Wert der Abgabeenergie-Bilanzmenge EB größer als eine minimale Abgabeenergie-Bilanzmenge EBMIN, insbesondere größer als null, ist. Ist dies der Fall, wird ein erstes Vergleichssignal A ausgegeben, und an das Einschaltmodul 323 geleitet. Dieses Vergleichssignal A, und auch die Vergleichssignale der weiteren Vergleichsmodule 322B, 322C, können insbesondere boolescher Natur sein, also ausschließlich den Wert null oder eins annehmen.

Ein Zyklusspeicher 325 speichert ein von dem Einschaltmodul 323 im vorherigen Zyklus Z -1 ausgegebenes Einschaltsignal D und stellt dieses im aktuellen Zyklus Z als vorheriges Einschaltsignal DV ausgangsseitig bereit. In einem zweiten Vergleichsmodul 322B, welches mit dem Zyklusspeicher 325 verbunden ist, wird geprüft, ob das vorherige Einschaltsignal DV positiv ist, das heißt DV = D(Z-1) = "on". Ist dies der Fall, wird ein positives zweites Vergleichssignal B ausgegeben und an das Einschaltmodul 323 geleitet.

In einem dritten Vergleichsmodul 322C wird die Abgabeenergie-Bilanzmenge EB mit dem Abgabeenergie-Grenzwert EBX verglichen. Ist die Abgabeenergie-Bilanzmenge EB größer als der Abgabeenergie-Grenzwert EBX, ist - im übertragenen Sinne - der Energiespeicher zu einem festgelegten Mindestwert gefüllt. In diesem Falle wird ein positives drittes Vergleichssignal C ausgegeben und an das Einschaltmodul 323 geleitet.

Das Einschaltmodul 323 gibt in Abhängigkeit der Vergleichssignale A , B, C ein Einschaltsignal D aus, welches den Wert "on" oder "off" annehmen kann. Gleichwohl kann alternativ oder zusätzlich vom Einschaltmodul 323 das Einschaltsignal in boolescher Form, also entsprechend 1 für "on" und 0 für "off", ausgegeben werden.

Fig. 4B zeigt mögliche Schaltzustände 323.1-5 eines Einschaltmoduls 323. In einem ersten Schaltzustand 323.1 ist das erste Vergleichssignal A negativ, das heißt A = 0. Hieraus resultiert, unabhängig davon ob das zweite Vergleichssignal B oder das dritte Vergleichssignal C positiv oder negativ sind, ein negatives Einschaltsignal D. Ein negatives Einschaltsignal D, die Schaltstellung "off", führt dazu, dass durch das Hochfrequenzgeneratormodul 120 keine elektrische Abgabeleistung PO abgegeben wird. Dieser Schaltzustand 323.1 bedeutet, dass die Abgabeenergie-Bilanzmenge EB zu dem aktuellen Zeitpunkt gleich null ist, und - im übertragenen Sinne - der virtuelle Energiespeicher leer ist.

In einem zweiten Schaltzustand 323.2 ist das erste Vergleichssignal A und das dritte Vergleichssignal C positiv, und das zweite Vergleichssignal B negativ, wodurch ein positives Einschaltsignal D, das heißt die Schaltstellung "on", resultiert. Dieser Schaltzustand 323.2 tritt ein, wenn die Abgabeenergie-Bilanzmenge EB im aktuellen Zyklus Z größer ist als der Abgabeenergie-Grenzwert EBX, und im vorherigen Zyklus Z-1 das Einschaltmodul 323 ein negatives Einschaltsignal D ausgegeben hat, folglich das Hochfrequenzgeneratormodul 120 im vorherigen Zyklus Z-1 keine Abgabeleistung PO abgegeben hat.

In einem dritten Schaltzustand 323.3 sind alle drei Vergleichssignale A, B, C positiv, was zu einem positiven Einschaltsignal D, das heißt der Schaltstellung "on" führt. Dieser Schaltzustand 323.3 bedeutet, dass die Abgabeenergie-Bilanzmenge EB zu dem aktuellen Zeitpunkt größer ist als der Abgabeenergie-Grenzwert EBX und im vorherigen Zyklus Z-1 das Einschaltmodul 323 ein positives Einschaltsignal D ausgegeben hat. Letzteres bedeutet, dass das Hochfrequenzgeneratormodul 120 im vorherigen Zyklus Z-1 eine Abgabeleistung PO abgegeben hat.

In einem vierten Schaltzustand 323.4 ist das erste Vergleichssignal A positiv, und das zweite Vergleichssignal B und das dritte Vergleichssignal C negativ, was zu einem negativen Einschaltsignal D, das heißt der Schaltstellung "off' führt. Dieser Schaltzustand 323.4 bedeutet, dass die Abgabeenergie-Bilanzmenge EB zu dem aktuellen Zeitpunkt kleiner ist als der Abgabeenergie-Grenzwert EBX und im vorherigen Zyklus Z-1 das Einschaltmodul 323 ein negatives Einschaltsignal D ausgegeben, das Hochfrequenzgeneratormodul 120 also keine Abgabeleistung PO abgegeben hat.

In einem fünften Schaltzustand 323.5 ist das erste Vergleichssignal A und das zweite Vergleichssignal B positiv, und das dritte Vergleichssignal C negativ, was zu einem positiven Einschaltsignal D, das heißt der Schaltstellung "on" führt. Dieser Schaltzustand 323.5 bedeutet, dass die Abgabeenergie-Bilanzmenge EB zu dem aktuellen Zeitpunkt sinkt, und kleiner ist als der Abgabeenergie-Grenzwert EBX und im vorherigen Zyklus Z-1 das Einschaltmodul 323 ein positives Einschaltsignal D ausgegeben hat, das Hochfrequenzgeneratormodul 120 also eine Abgabeleistung PO abgegeben hat.

Im Zusammenhang mit den Schaltzuständen sind insbesondere der vierte Schaltzustand 323.4 und der fünfte Schaltzustand 323.5 zu erwähnen: In beiden Schaltzuständen 323.4, 323.5 ist die Abgabeenergie-Bilanzmenge EB kleiner als der Abgabeenergie-Grenzwert EBX.

Im vierten Schaltzustand 323.4 war dabei das vorherige Einschaltsignal DV, also das Einschaltsignal D in einem vorherigen Zyklus Z-1, negativ, das heißt es wurde keine Abgabeenergie PO durch das Hochfrequenzgeneratormodul 120 abgegeben.

Wenn dabei jedoch, wie im fünften Schaltzustand 323.5, das Einschaltsignal D in einem vorherigen Zyklus Z-1 positiv war, das heißt das Hochfrequenzgeneratormodul 120 eine Abgabeenergie PO abgegeben hat, gibt das Hochfrequenzgeneratormodul 120 auch weiter - aufgrund der Ausgabe eines positiven Einschaltsignals D durch das Einschaltmodul 323 in einem aktuellen Zyklus Z - ein Abgabeenergie PO ab. Vereinfacht ausgedrückt: ein aktiviertes Hochfrequenzgeneratormodul 120 bleibt also aktiviert - auch wenn die Abgabeenergie-Bilanzmenge EB den Abgabeenergie-Grenzwert EBX unterschreitet - und wird erst deaktiviert, wenn die Abgabeenergie-Bilanzmenge EB die minimale Abgabeenergie-Bilanzmenge EBMIN unterschreitet, insbesondere gleich null ist. "Aktiviert" und "deaktiviert" bezieht sich hierbei insbesondere auf die Abgabe einer elektrischen Abgabeenergie PO über den Ausgang 120.2 des Hochfrequenzgeneratormodul 120, und nicht, dass das Netzteil 110 und/oder der gesamte Hochfrequenzgenerator 10 eingeschaltet bzw. abgeschaltet wird.

Liste der Bezugszeichen

| | |
|---|---|
| 10 | Hochfrequenzgenerator |
| 110 | Netzteil, taktsteuerbares Netzteil |
| 110.1 | Eingang des Netzteils |
| 110.2 | Ausgang des Netzteils |
| 120 | Hochfrequenzgeneratormodul |
| 122 | Antifaradisationskondensator |
| 125 | Ausgangsanschluss, Ausgangspole des Hochfrequenzgenerators |
| 125.1 | Erster Ausgangspol |
| 125.2 | Zweiter Ausgangspol |
| 205 | Elektrochirurgisches Instrument |
| 210 | Schneidelektrode, Bipolare Elektrode des elektrochirurgischen Instruments |
| 212 | Griffteil des elektrochirurgischen Instruments |
| 230 | Gewebe |
| 232 | Kochsalzlösung, Saline |
| 234 | Plasma |
| 240 | Fußschalter |
| 300 | Leistungssteuerung |
| 320 | Bilanzierungseinheit |
| 322 | Aktivator |
| 322A | Erstes Vergleichsmodul |
| 322B | Zweites Vergleichsmodul |
| 322C | Drittes Vergleichsmodul |
| 323 | Einschaltmodul |
| 323.1-5 | Erster bis fünfter Schaltzustand |
| 325 | Zyklusspeicher |
| 330 | Leistungserfassungseinheit |
| 332 | Erster Integrator |
| 334 | Zweiter Integrator |
| 336 | Summenbilder |
| 338 | Differenzbilder |
| 342 | Generatorleistung-Einstellmodul |
| 344 | Initialenergie-Einstellmodul |
| 346 | Grenzwert-Einstellmodul |
| 348 | Multiplikator |
| 356 | Zündspannung |
| 610 | Virtueller Energiespeicher |
| 630 | Tatsächliche Energiemenge |
| A, B, C | Erstes bis drittes Vergleichssignal |
| D | Einschaltsignal |
| DV | Vorheriges Einschaltsignal |
| EA | Zulässige Energiemenge |
| EAV | Abgegebene Energiemenge |
| EB | Abgabeenergie-Bilanzmenge |
| EBMAX | Maximale Abgabeenergie-Bilanzmenge |
| EBMIN | Minimale Abgabeenergie-Bilanzmenge, Minimalwert |
| EBX | Abgabeenergie-Grenzwert |
| EO | Abgabe-Energiemenge |
| ES | Initialenergie |
| HFOFF | Deaktivierter Zustand des Hochfrequenzgenerators |
| HFON | Aktivierter Zustand des Hochfrequenzgenerators |
| PAV | Vorgegebene Generatorleistung |
| PC | Schneidleistung |
| PI | Zündleistung |
| PO | Abgabeleistung, elektrische Leistung |
| T | Zeit |
| TA | Bestimmungsdauer |
| TB | Bilanzdauer |
| TI | Zündzeitpunkt |
| TZ | Zykluszeit |
| Z | Zyklus, Berechnungszyklus |

**Patentansprüche**

1. Hochfrequenzgenerator (10) zum Anschließen eines elektrochirurgischen Instrumentes (205) mit

- einem elektrischen Ausgangsanschluss (125) für ein elektrochirurgisches Instrument (205),
- einem Netzteil (110), das wenigstens mittelbar mit dem Ausgangsanschluss (125) verbunden ist,
- einer Leistungssteuerung (300) zum Steuern der über den Ausgangsanschluss (125) abgegebenen elektrischen Abgabeleistung (PO),

**dadurch gekennzeichnet, dass**
die Leistungssteuerung (300) ausgebildet ist,

- die Abgabe einer elektrischen Abgabeleistung (PO) zu beginnen, wenn eine Abgabeenergie-Bilanzmenge (EB) größer als ein Abgabeenergie-Grenzwert (EBX) ist, und
- die Abgabe der elektrischen Abgabeleistung (PO) zu beenden, wenn die Abgabeenergie-Bilanzmenge (EB) einen Minimalwert (EBMIN) unterschreitet, wobei
- die Abgabeenergie-Bilanzmenge (EB) über eine gleitende Bestimmungsdauer (TA) aus einer zugeführten vorgegebenen Generatorleistung (PAV) und der abgegebenen elektrischen Abgabeleistung (PO) bestimmt wird.

2. Hochfrequenzgenerator (10) nach Anspruch 1, **da-**

**durch gekennzeichnet, dass** die vorgegebene Generatorleistung (PAV) einstellbar ist.

3. Hochfrequenzgenerator (10) nach Anspruch 1 oder 2, **dadurch gekennzeichnet, dass** die Abgabeenergie-Bilanzmenge (EB) eine maximale Abgabeenergie-Bilanzmenge (EBMAX) nicht überschreitet.

4. Hochfrequenzgenerator (10) nach Anspruch 3, **dadurch gekennzeichnet, dass** die maximale Abgabeenergie-Bilanzmenge (EBMAX) aus der Differenz aus einer zulässigen Energiemenge (EA) und einer vorgegebenen Energiemenge (EAV) bestimmt wird, wobei die zulässige Energiemenge (EA) die Energiemenge ist, die in einer Bilanzdauer (TB) maximal abgegeben werden darf, und sich die vorgegebene Energiemenge (EAV) aus der vorgegebenen Generatorleistung (PAV) in der Bilanzdauer (TB) bestimmt.

5. Hochfrequenzgenerator (10) nach mindestens einem der vorherigen Ansprüche, **dadurch gekennzeichnet, dass** der Abgabeenergie-Grenzwert (EBX) einstellbar ist.

6. Hochfrequenzgenerator (10) nach mindestens einem der vorherigen Ansprüche, **dadurch gekennzeichnet, dass** die Leistungssteuerung (300) eine Bilanzierungseinheit (320) aufweist, die ausgebildet ist zum zyklischen Bestimmen der Abgabeenergie-Bilanzmenge (EB).

7. Leistungssteuerung (300) für einen Hochfrequenzgenerator (10) nach einem der vorherigen Ansprüche, wobei der Hochfrequenzgenerator (10) aufweist:

- einen elektrischen Ausgangsanschluss (125) für ein elektrochirurgisches Instrument (205),
- eine Strom- oder Spannungsquelle (110), die wenigstens mittelbar mit dem Ausgangsanschluss (125) verbunden ist, und
- eine Leistungssteuerung (300),

**dadurch gekennzeichnet, dass** die Leistungssteuerung (300) ausgebildet ist,

- die Abgabe einer elektrischen Abgabeleistung (PO) zu beginnen, wenn eine Abgabeenergie-Bilanzmenge (EB) größer als ein Abgabeenergie-Grenzwert (EBX) ist oder einen Initialwert (EBI) aufweist, und
- die Abgabe der elektrischen Abgabeleistung (PO) zu beenden, wenn die Abgabeenergie-Bilanzmenge (EB) einen Minimalwert (EBMIN) unterschreitet, wobei
- die Abgabeenergie-Bilanzmenge (EB) über eine gleitende Bestimmungsdauer (TA) bestimmt wird, wobei die Abgabeenergie-Bilanzmenge (EB) als eine Differenz aus den Integralen einer vorgegebenen Generatorleistung (PAV) und der elektrischen Abgabeleistung (PO) gebildet wird.

**Claims**

1. A high-frequency generator (10) for connecting an electrosurgical instrument (205), comprising

- an electrical output connection point (125) for an electrosurgical instrument (205),
- a power supply (110), which is at least indirectly connected to the output connection point (125),
- a power controller (300) for controlling the electrical output power (PO) output via the output connection point (125),

**characterized in that** the power controller (300) is configured

- to begin the output of an electrical output power (PO) when an output energy balance amount (EB) is greater than an output energy limit value (EBX) and
- to end the output of the electrical output power (PO) when the output energy balance amount (EB) falls below a minimum value (EBMIN),
- the output energy balance amount (EB) being determined over a moving determination duration (TA) from a supplied specified generator power (PAV) and the output electrical output power (PO).

2. A high-frequency generator (10) according to claim 1, **characterized in that** the specified generator power (PAV) is adjustable.

3. A high-frequency generator (10) according to claim 1 or 2, **characterized in that** the output energy balance amount (EB) does not exceed a maximum output energy balance amount (EBMAX).

4. A high-frequency generator (10) according to claim 3, **characterized in that** the maximum output energy balance amount (EBMAX) is determined from the difference between a permissible energy amount (EA) and a specified energy amount (EAV), wherein the permissible energy amount (EA) is the maximum energy amount that may be output during a balance duration (TB), and the specified energy amount (EAV) is determined from the specified generator power (PAV) during the balance duration (TB).

5. A high-frequency generator (10) according to at least one of the previous claims, **characterized in that** the output energy limit value (EBX) is adjustable.

6. A high-frequency generator (10) according to at least one of the previous claims, **characterized in that** the power controller (300) comprises a balancing unit (320), which is configured to cyclically determine the output energy balance amount (EB).

7. A power controller (300) for a high-frequency generator (10) according to one of the previous claims, wherein the high-frequency generator (10) comprises:

- an electrical output connection point (125) for an electrosurgical instrument (205),
- a current or voltage source (110), which is at least indirectly connected to the connection output point (125), and
- a power controller (300),

**characterized in that** the power controller (300) is configured

- to begin the output of an electrical output power (PO) when an output energy balance amount (EB) is greater than an output energy limit value (EBX) or has an initial value (EBI), and
- to end the output of the electrical output power (PO) when the output energy balance amount (EB) falls below a minimum value (EBMIN),
- the output energy balance amount (EB) being determined over a moving determination duration (TA), wherein the output energy balance amount (EB) is formed as a difference between the integrals of a specified generator power (PAV) and the electrical output power (PO).

**Revendications**

1. Générateur haute fréquence (10) pour le raccordement d'un instrument électrochirurgical (205) avec

- une borne de sortie (125) électrique pour un instrument électrochirurgical (205),
- un bloc d'alimentation (110), qui est relié au moins indirectement à la borne de sortie (125),
- une commande de puissance (300) pour la commande de la puissance de distribution électrique (PO) distribuée par l'intermédiaire de la borne de sortie (125),

**caractérisé en ce que**
la commande de puissance (300) est réalisée

- pour commencer la distribution d'une puissance de distribution électrique (PO), lorsqu'une quantité d'équilibre d'énergie distribuée (EB) est supérieure à une valeur limite d'énergie distribuée (EBX), et
- pour mettre fin à la distribution de la puissance de distribution électrique (PO), lorsque la quantité d'équilibre d'énergie distribuée (EB) passe au-dessous d'une valeur minimale (EBMIN), dans lequel
- la quantité d'équilibre d'énergie distribuée (EB) est déterminée pendant une durée de détermination (TA) glissante à partir d'une puissance de générateur prédéfinie (PAV) amenée et de la puissance de distribution électrique (PO) distribuée.

2. Générateur haute fréquence (10) selon la revendication 1, **caractérisé en ce que** la puissance de générateur prédéfinie (PAV) est réglable.

3. Générateur haute fréquence (10) selon la revendication 1 ou 2, **caractérisé en ce que** la quantité d'équilibre d'énergie distribuée (EB) ne dépasse pas une quantité d'équilibre d'énergie distribuée maximale (EBMAX).

4. Générateur haute fréquence (10) selon la revendication 3, **caractérisé en ce que** la quantité d'équilibre d'énergie distribuée maximale (EBMAX) est déterminée à partir de la différence d'une quantité d'énergie admissible (EA) et d'une quantité d'énergie prédéfinie (EAV), dans lequel la quantité d'énergie admissible (EA) est la quantité d'énergie qui peut être distribuée au maximum dans une durée d'équilibre, et la quantité d'énergie prédéfinie (EAV) se détermine à partir de la puissance de générateur prédéfinie (PAV) dans la durée d'équilibre (TB).

5. Générateur haute fréquence (10) selon au moins l'une des revendications précédentes, **caractérisé en ce que** la valeur limite d'énergie distribuée (EBX) est réglable.

6. Générateur haute fréquence (10) selon au moins l'une des revendications précédentes, **caractérisé en ce que** la commande de puissance (300) présente une unité d'équilibrage (320), qui est réalisée pour la détermination cyclique de la quantité d'équilibre d'énergie distribuée (EB).

7. Commande de puissance (300) pour un générateur haute fréquence (10) selon l'une quelconque des revendications précédentes, dans laquelle le générateur haute fréquence (10) présente :

- une borne de sortie (125) électrique pour un instrument électrochirurgical (205),
- une source de courant ou de tension (110), qui est reliée au moins indirectement à la borne de sortie (125), et
- une commande de puissance (300),

**caractérisée en ce que** la commande de puissance (300) est réalisée

- pour commencer la distribution d'une puissance de distribution électrique (PO), lorsqu'une quantité d'équilibre d'énergie distribuée (EB) est supérieure à une valeur limite d'énergie distribuée (EBX) ou présente une valeur initiale (EBI), et
- pour mettre fin à la distribution de la puissance de distribution électrique (PO), lorsque la quantité d'équilibre d'énergie distribuée (EB) passe au-dessous d'une valeur minimale (EBMIN), dans laquelle
- la quantité d'équilibre d'énergie distribuée (EB) est déterminée par l'intermédiaire d'une durée de détermination (TA) glissante, dans laquelle la quantité d'équilibre d'énergie distribuée (EB) est formée en tant qu'une différence des intégrales d'une puissance de générateur prédéfinie (PAV) et de la puissance de distribution électrique (PO).

Fig. 1

Fig. 2

Fig. 3A

Fig. 3B

Fig. 3C

EP 3 764 930 B1

**Fig. 4A**

| | A | B | C | ⇒ | D |
|---|---|---|---|---|---|
| 323.1 | 0 | 0/1 | 0/1 | | off |
| 323.2 | 1 | 0 | 1 | | on |
| 323.3 | 1 | 1 | 1 | | on |
| 323.4 | 1 | 0 | 0 | | off |
| 323.5 | 1 | 1 | 0 | | on |

**Fig. 4B**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010108523 A
- WO 9509577 A1